# EUROPÄISCHE PATENTSCHRIFT

(11) **EP 3 328 505 B1**
(45) Veröffentlichungstag und Bekanntmachung des Hinweises auf die Patenterteilung: **04.03.2020**
(21) Anmeldenummer: 16736497.5
(22) Anmeldetag: 11.07.2016
(51) Int. Cl.: A61Q 15/00, A61K 8/25, A61K 8/19

(54) **ANTITRANSPIRANT**
ANTIPERSPIRANT
ANTISUDORAL

(30) Priorität: 27.07.2015 DE 102015214146
(43) Veröffentlichungstag der Anmeldung: 06.06.2018
(73) Patentinhaber: Beiersdorf AG, 20253 Hamburg (DE)
(72) Erfinder: GROTHEER, Elke, 25474 Bönningstedt (DE); STÄB, Franz, 21379 Echem (DE); SCHMIDT-ROSE, Thomas, 22605 Hamburg (DE); SEET, Michael, 68163 Mannheim (DE); KLAUCK, Robert, 21465 Reinbek (DE); LEHMBECK, Frank, 22848 Norderstedt (DE)
(86) Internationale Anmeldenummer: PCT/EP2016/066396
(87) Internationale Veröffentlichungsnummer: WO 2017/016861

(56) Entgegenhaltungen:
- EP-A1- 1 026 093
- EP-A2- 0 337 464
- WO-A1-2014/191236
- CH-A- 470 183
- DE-A1-102005 059 935
- FR-A1- 2 977 151
- US-A- 5 884 759
- US-A1- 2007 148 113
- US-B1- 6 474 861

## Beschreibung

Die vorliegende Erfindung betrifft ein Kosmetikum zur Verminderung oder Verhinderung der apoekkrinen Schweißbildung aus einem Packmittel mit zwei Vorratskammern und zwei kosmetischen Teilzubereitungen, dadurch gekennzeichnet, dass eine Kammer eine Teilzubereitung, enthaltend niedermolekulare, hoch amorphe Kieselsäure in Kombination mit ein oder mehreren Stabilisatoren, beinhaltet und die zweite Kammer eine Zubereitung, enthaltend mindestens eine Base, beinhaltet.

Als Schweiß wird ein von der Haut des Menschen über so genannte Schweißdrüsen abgesondertes wässriges Sekret bezeichnet. Es gibt drei Arten von Schweißdrüsen in der Haut, nämlich apokrine, ekkrine und apoekkrine Schweißdrüsen (Int J Cosmet Sci. 2007 Jun; 29(3):169-79).

Die ekkrinen Schweißdrüsen sind beim Menschen praktisch über den ganzen Körper verteilt und können beträchtliche Mengen eines klaren, geruchlosen Sekretes produzieren, das zu über 99% aus Wasser besteht. Im Gegensatz dazu kommen die apokrinen Schweißdrüsen nur in den behaarten Körperarealen der Achsel- und Genitalregion sowie an den Brustwarzen vor. Sie produzieren geringe Mengen eines milchigen Sekretes, das Proteine und Lipide enthält und chemisch neutral ist.

Das Schwitzen, auch als Transpiration bezeichnet, ist ein effektiver Mechanismus, um überschüssige Wärme abzugeben und damit die Körpertemperatur zu regulieren. Hierzu dient vor allem das volumenreiche wässrige Sekret der ekkrinen Drüsen, die beim Erwachsenen bis zu 2-4 Liter pro Stunde bzw. 10-14 Liter am Tag produzieren können.

Dem Schweiß - insbesondere dem Sekret der apokrinen Schweißdrüsen - wird darüber hinaus eine Signalwirkung über den Geruchssinn zugesprochen. Beim Menschen spielt der apokrine Schweiß insbesondere im Zusammenhang mit dem emotionalen oder stressbedingten Schwitzen eine Rolle.

Kosmetische Antitranspirantien oder Desodorantien/Deodorantien dienen dazu, Körpergeruch zu beseitigen bzw. deren Entstehung zu vermindern. Körpergeruch entsteht, wenn der an sich geruchlose frische Schweiß durch Mikroorganismen wie z.B. Staphylokokken und Corynebakterien zersetzt wird.

Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt.

Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruchs (Geruchsverbesserungsmittel). Den üblichen kosmetischen Desodorantien liegen unterschiedliche Wirkprinzipien zugrunde.

Gängige Wirkmechanismen hierzu sind antibakterielle Effekte, wie sie z.B. auch das nichtkolloidale Silber zeigt, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Reaktionen zu wohlriechenden Stoffen umgesetzt werden.

Schweißgeruch besteht zu einem Großteil aus verzweigt-kettigen Fettsäuren, die durch bakterielle Enzyme aus geruchlosem Schweiß freigesetzt werden. Klassische Deo-Wirkstoffe wirken dem entgegen, indem sie das Wachstum von Bakterien reduzieren. Häufig wirken die dabei zum Einsatz kommenden Substanzen jedoch unselektiv auch gegen nützliche Hautkeime und können bei empfindlichen Personen zu Hautirritationen führen.

Als klassische Antitranspirantien werden vor allem Aluminiumsalze oder Aluminium-/Zirkonium-Salze verwendet. Diese hemmen den Schweißfluss durch Verstopfung der Ausführungsgänge der Schweißdrüsen, indem sie vor Ort zusammen mit hauteigenen Proteinen ausfallen und so zu sogenannten Plugs führen. Daher kann es zu einem Stau des Schweißes innerhalb der Drüse kommen.

Die Wirkung von Antitranspirantien auf Basis von Al-Salzen gegen thermisches Schwitzen unter normalen physiologischen Bedingungen ist sehr gut untersucht.

Ob diese Verstopfung durch Denaturierung des Keratins oder durch Verklumpung von Korneozyten im Schweißdrüsengang verursacht wird (Shelley WB and Hurley HJ, Acta. Derm. Venereol. (1975) 55: 241-60), oder durch die Entstehung eines ACH/AZG-Gels (Reller HH and Luedders WL, in: Advances in Modern Toxicology, Dermatoxicology and Pharmocology, F.N. Marzulli and HI. Maibach, Eds. Hemisphere Publishing Company, Washington and London (1977) Vol. 4: 1-5), das durch Neutralisation im Schweißdrüsenausführgang gebildet wird, ist nach wie vor offen.

Die so erzielte und bekannte Verstopfung ist allerdings nur kurzfristig wirksam. Starkes Schwitzen oder die Reinigung der Achsel im Rahmen der normalen Körperreinigungsroutine heben die Verstopfung wieder auf und somit auch den Antitranspiranteffekt. Die daraus resultierende Notwendigkeit, Antitranspirant (AT)-Produkte mindestens einmal täglich aufzutragen führt aber ggf. zu Hautreizungen, speziell nach der Rasur oder in oder an vorgeschädigten Hautarealen.

Darüber hinaus können solche Aluminiumsalze wie Aluminiumhydroxychloride bei häufiger Anwendung und empfindlichen Personen Hautschäden hervorrufen. Darüber hinaus kann es durch den Einsatz der Aluminiumsalze zu Verfärbungen von Textilien kommen, die mit dem Antitranspirans in Kontakt kommen.

Durch die zusätzliche Verwendung antimikrobieller Stoffe in kosmetischen Antitranspirantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt.

Üblicherweise werden Antitranspirantien (AT) und Deodorantien (Deo) in mannigfaltigen Produktformen angeboten, wobei in Europa Roller, Pumpzerstäuber und Aerosole dominieren, in den USA, Mittel- und Südamerika eher die Deo-Stifte ("Sticks"). Es sind sowohl wasserfreie (Suspensionen) als auch wasserhaltige Produkte (hydro-alkoholische Formulierungen, Emulsionen) bekannt.

An ein zufriedenstellendes Deo-Mittel werden folgende Voraussetzungen geknüpft: 1) Schonung der natürlichen Biologie der Haut 2) Duftneutralität 3) Wirksamkeit nur in Bezug auf Desodorierung, d.h. nur Vermeidung und/oder Beseitigung von Körpergeruch 4) Vermeidung der Bildung von resistenten Bakterienstämmen 5) Vermeidung der Akkumulation der Wirkstoffe auf der Haut 6) Unschädlichkeit bei Überdosierung oder sonstiger nicht bestimmungsgemäßer Anwendung 7) Gute kosmetische Anwendung 8) Leichte Handhabung (z.B. als Flüssigkeit) und universelle Verwendbarkeit in verschiedensten kosmetischen und externen Zubereitungen. 9) Ausgezeichnete Haut- und Schleimhautverträglichkeit 10) Einsatz umweltfreundlicher Stoffe

Bekannt und gebräuchlich sind neben den flüssigen Desodorantien und Antitranspirantien auch feste Zubereitungen, beispielsweise Puder, Pudersprays als, Intimreinigungsmittel.

Die Art der Zusammensetzung kosmetischer Zubereitungen findet ihre natürlichen Grenzen, die durch die Verträglichkeit der Einzelkomponenten miteinander sowie der Stabilität von Einzelkomponenten im Trägermedium bestimmt werden. Auch die Kombination unterschiedlich geladener Polymerer oder Tenside führt in kosmetischen Formulierungen zu Verklumpung und Ausfällungen.

Es hat nicht an Versuchen gefehlt, derartige Zubereitungen dem Verbraucher zugänglich zu machen. Meist werden dann Teilkomponenten der Formulierungen getrennt verpackt und aufbewahrt. In der Regel werden hierzu sogenannte Zwei-Kammer-Packmittel (engl. dual chamber) verwendet, bei denen die Teilzubereitungen in getrennten Vorratsgefäßen aufbewahrt und dem Packungsbehältnis gleichzeitig über eine gemeinsame Öffnung entnommen werden können.

EP 1026093 offenbart ein Zweikammerpackmittel, bei dem die Kammern durch eine mechanisch entfernbare Barriere voneinander getrennt wurden. Vor der Applikation wird die Barriere (Stopfen) entfernt und der Inhalt der beiden Kammern kann gemischt werden.

In EP 462255 und EP 1044893 wird ein Zweikammerpackmittel offenbart, bei dem die Barriere zwischen den Kammern vor dem ersten Betätigen der Sprühvorrichtung entfernt wird und somit die Mischung aus den Kammerinhalten appliziert wird.

EP 0816253 offenbart ein Zweikammerpackmittel, bei dem durch Druck auf die eine Kammer ein die Kammern trennendes Diaphragma durchstoßen wird und der Inhalt der einen Kammer in die zweite Kammer gelangt und mit dessen Inhalt durch Schütteln gemischt werden kann.

Im der Deodorant- und/oder Antitranspirantprodukte ist die Verwendung von Zweikammerpackmitteln noch unbekannt, da die bisher eingesetzten aluminiumhaltigen Formulierungen, in denen ACH als Feststoff vorliegt, außerordentlich stabil sind.

Nachteilig an den bislang zur Schweißhemmung verwendeten Aluminium-Salzen ist, die derzeit noch nicht vollständig geklärte Langzeittoxizität. Aluminium steht seit langem im Verdacht neurodegenarative Krankheiten wie Demenz, insbesondere Alzheimer, zu fördern oder auszulösen. Auch steht wird Aluminium mit der Entstehung von Brustkrebs in Verbindung gebracht. Bisher gibt es keine gesicherten Nachweise, dass über die Haut wirkende aluminiumhaltige AT-Mittel daran beteiligt sind. Bei intakter Haut sind die maximal zulässigen Aufnahmemengen nicht zu erreichen.

In Hinblick auf die Datenlage ist jedoch die Abkehr von aluminumhaltigen AT-Mitteln von Vorteil, so dass die Industrie händeringend nach aluminiumfreien Alternativen sucht.

Aufgrund dieser Problemstellung ist es wünschenswert Produkte zur Verfügung zu stellen, die eine Antitranspirantwirkung ohne Verwendung von Al-Salzen erzielen.

Eine Alternative zu Al-Salzen stellen kurzkettige Silikate in Form von Kieselsäuren dar, die eine Schweißbildung zuverlässig unterdrücken können.

Als Kieselsäuren werden die Sauerstoffsäuren des Siliciums bezeichnet. Die einfachste Kieselsäure ist Monokieselsäure (Orthokieselsäure) Si(OH)₄. Sie ist eine schwache Säure (pKs1 = 9,51; pKs2 = 11,74) und neigt zur Kondensation. Wasserabspaltungen führen zu Verbindungen wie Dikieselsäure (Pyrokieselsäure) (HO)₃Si-O-Si(OH)₃ und Trikieselsäure (HO)₃Si-O-Si(OH)₂-O-Si(OH)₃. Cyclische (ringförmige) Kieselsäuren sind z. B. Cyclotrikieselsäure und Cyclotetrakieselsäure mit der allgemeinen Summenformel [Si(OH)₂-O-]ₙ. Polymere werden gelegentlich als Metakieselsäure (H₂SiO₃, [-Si(OH)₂-O-]ₙ) bezeichnet. Kondensieren diese niedermolekularen Kieselsäuren weiter, bilden sich amorphe Kolloide (Kieselsol). Allgemeine Summenformel aller Kieselsäuren ist H₂ₙ+2SiₙO₃ₙ₊₁. Als Summenformel wird häufig SiO₂ · nH₂O angegeben; das Wasser ist bei Kieselsäuren jedoch kein Kristallwasser, sondern kann nur durch eine chemische Reaktion abgespalten werden und bildet sich aus konstitutionell gebundenen Hydroxygruppen.

Allgemein werden die wasserärmeren Produkte der Orthokieselsäure unter dem Begriff Polykieselsäuren zusammengefasst. Formales Endprodukt der Wasserabspaltung ist Siliciumdioxid, das Anhydrid der Kieselsäure. Die Salze der Säuren nennt man Silicate. Technisch verwendete bzw. hergestellte Alkalisalze werden oft Wassergläser genannt. Die Ester der Kieselsäuren werden Kieselsäureester genannt.

Als niedermolekulare, hoch amorphe Kieselsäuren sind im Sinne der Erfindung nur die Kieselsäuren zu verstehen, die eine Ausdehnung von 1 bis 100 nm, gemessen durch dynamische Lichtstreuung, aufweisen. Diese niedermolaren, hoch amorphen Kiselsäuren werden sind auch unter der Bezeichnung niedermolekulare Polykieselsäuren bekannt und werden im Folgenden als NPK bezeichnet.

NPK lassen sich wie folgt darstellen:
a) Herstellung einer alkalischen Silikatlösung mit einem pH-Wert >= 10
b) Erniedrigung des pH-Wertes durch Zugabe einer Säure auf einen pH-Wert <= 1, wobei die Polykieselsäure entsteht und wobei die Erniedrigung des pH-Wertes innerhalb von weniger als 60 Sekunden erfolgt
c) Erhöhung des pH-Wertes durch Zugabe von Basen

Die nach diesem Verfahren hergestellten Kieselsäuren sind jedoch nur bei diesen sehr niedrigem pH-Werten stabil. Ab einem pH-Wert von 3,5 erfolgt eine Kondensation, was sich durch Ausfallen Gelbildung bemerkbar macht. Diese Niederschläge aus hochmolekularen Kieselsäuren haben keine AT-Wirkung mehr.

Ein Nachteil der so hergestellten NPK ist deren Unverträglichkeit mit vielen der üblichen Kosmetischen Hilfsstoffe wie Emulgatoren, Tenside und Ölen. Dadurch ist es nahezu nicht möglich die üblichen Formulierungsformen wie Emulsionen stabil herzustellen.

Da pH-Werte unter 3,5 physiologisch unverträglich sind, müssen Zubereitungsformen gefunden werden, bei denen der pH-Wert mindestens bei 3,5 liegt und bei denen keine Gelbildung bzw. Ausfällung amorpher Kolloide (Kieselsol) erfolgt.

Wünschenswert wäre es demnach ein Antitranspirantprodukt zur Verfügung zu stellen, das die vorgenannten Nachteile und Nebenwirkungen, insbesondere der ACH-haltigen Zubereitungen, nicht aufzeigt.

Wünschenswert wäre es weiterhin, ein Antitranspirantprodukt zur Verfügung zu stellen, das den Stand der Technik bereichert und eine Alternative zu den bekannten Zubereitungen, insbesondere ACH-haltigen Zubereitungen, darstellt.

Insbesondere ist es auch wünschenswert ein Antitranspirantprodukt zur Verfügung zu stellen, das eine möglichst breite Möglichkeiten der galenischen Formulierung in kosmetisch akzeptable und attraktive Formelsysteme ermöglicht.

Eine weitere Aufgabe der vorliegenden Erfindung war es also, ein Produkt zu entwickeln, welche als Grundlage für kosmetische Desodorantien bzw. Antitranspirantien geeignet sind, und die Nachteile des Standes der Technik nicht aufweisen, insbesondere sich durch gute Hautverträglichkeit auszeichnen.

Aufgabe der Erfindung ist es insbesondere, kieselsäurehaltige Antitranspirantzubereitungen enthaltend niedermolekulare Polykieselsäuren (NPK) in Kombination mit ein oder mehreren Stabilisatoren zur Verfügung zu stellen, bei ausreichender Stabilität einen physiologisch verträglichen pH-Wert aufweisen.

Es war nach all diesem überraschend und nicht vorhersehbar, dass kieselsäurehaltige Zubereitungen mit einem pH von mindestens 3,5 , enthaltend NPK und ein oder mehrere Stabilisatoren zur Verwendung als hautverträgliche Antitranspirantien, also zur Verminderung oder Verhinderung der apoekkrinen Schweißbildung, eignen, wenn die pH-Werterhöhung erst kurz vor der Applikation erfolgt und dadurch die Nachteile des Standes der Technik, die mangelnde Stabilität von physiologisch verträglichen NPK-Zubereitungen, beseitigt wird.

Es war erstaunlich, dass mittels getrennter Bereitstellung von einer NPK-haltiger Zubereitung und einer Base in einem Zweikammerpackmittel, wobei die NPK-haltige Zubereitung mit der Base erst kurz vor oder während der Ausgabe und/oder Applikation gemischt werden, nicht nur für kosmetische Zwecke hervorragend geeignet sind, sondern überdies wirkungsvoller und schonender sind als die Verwendung von stabilen Zusammensetzungen des Standes der Technik.

Die Erfindung ist daher ein kosmetisches Produkt aufweisend ein Zweikammerbehältnis geeignet zur Applikation von kosmetischen Zubereitungen, dadurch gekennzeichnet, dass eine Kammer eine Teilzubereitung, enthaltend niedermolekulare, hoch amorphe Kieselsäure (NPK) in Kombination mit ein oder mehreren Stabilisatoren, beinhaltet (Phase 1) und die zweite Kammer eine Zubereitung, enthaltend mindestens eine Base, beinhaltet (Phase 2), wobei erste und zweite Kammer durch eine Barriere voneinander getrennt sind, wobei die Barriere durch Einwirkung von Kräften auf die zweite Kammer zerstört wird, wodurch der Inhalt der zweiten Kammer in die erste Kammer übertreten kann und sich Inhalt der ersten Kammer und Inhalt der zweiten Kammer vermischen und anschließend die Mischung entnommen werden kann.

Die Erfindung umfasst demnach die Verwendung von NPK, als Antitranspirantwirkstoff, bevorzugt in topisch applizierbaren, insbesondere kosmetischen und/oder dermatologischen, Zubereitungen, wobei der pH-Wert der Zubereitung erst vor der Applikation auf ein physiologisch verträgliches Maß eingestellt wird, insbesondere der pH-Wert der applizierten Zubereitung nicht kleiner als 3,5 ist.

Durch die pH-Werterhöhung erst vor der Verwendung, kann die Lagerstabilität der NPK-haltigen Zubereitung vor der Verwendung sichergestellt werden.

Die Stabilisatoren werden gewählt aus
der Gruppe A:
   Hexenol cis 3 (CAS 928-96-1), Terpineol (CAS 8000-41-7), Linalool (CAS 78-70-6), Tetrahydrolinalool (CAS 78-69-3), Triethyl Citrate (CAS 77-93-0), 2-Isobutyl-4-hydroxy-4-methyltetrahydropyran (CAS 63500-71-0), Hexyl salicylate (CAS 6259-76-3), Phenylethyl alcohol (CAS 60-12-8), 3-Methyl-5-phenyl-1-pentanol (CAS 55066-48-3), 2,6-Dimethyl-7-octen-2-ol (CAS 18479-58-8), Benzyl salicylate (CAS 118-58-1), Geraniol (CAS 106-24-1), Citronellol (CAS 106-22-9) und Ethyllinalool (CAS 10339-55-6);
der Gruppe B: Alkohole und Diole
und der Gruppe C: Substanzen mit mindestens drei Hydroxylgruppen.

Insbesondere vorteilhaft sind Stabilisatoren aus der Gruppe Linalool (CAS 78-70-6), Benzyl salicylate (CAS 118-58-1), Geraniol (CAS 106-24-1) und Citronellol (CAS 106-22-9).

Insbesondere vorteilhafte Stabilisatoren aus der Gruppe B sind: Ethanol, 2-Propanol, PEG 8, Triethylenglycol, Methylphenylbutanol, Decandiol, Polyglyceryl-2-caprate, Oxalsäure.

Insbesondere vorteilhafte Stabilisatoren aus der Gruppe C sind: Sucrose (Mannose, Mannit), Glycerin, Pentaerithritol, Threitol, Erythritol, Hyaluronsäure.

Die topische Applikation von Zubereitungen - also die Verwendung von kosmetischen oder dermatologischen Zubereitungen auf der Haut - enthaltend NPK in Kombination mit ein oder mehreren Stabilisatoren gewählt aus den Gruppe A, B und/oder C ermöglicht die Verminderung oder Verhinderung des Stress-Schwitzens.

Im Rahmen der Erfindung, wird als Antitranspirantwirkung die Möglichkeit der Verminderung oder Verhinderung der Schweißbildung verstanden. D.h. NPK wirken als Schweißhemmer und vermindern die Schweißbildung und damit mittelbar auch den Schweißgeruch.

Durch die pH-Werterhöhung der NPK-haltigen Zubereitung vor der Ausbringung und Applikation, werden Zubereitungen zur Verfügung gestellt, die eine hohe physiologische Verträglichkeit aufweisen.

Die Mischung aus NPK-haltigen Zubereitung mit der pH-Wert erhöhenden Base erst vor der Ausbringung aus einem Zweikammerpackmittel macht die Verwendung von NPK-haltigen Zubereitungen als verträgliches Antitranspirantmittel erst möglich.

Erfindungsgemäße Produkte mit NPK, ermöglichen eine schweißhemmende Wirkung in der Größenordnung wie bekannte und bewährte Antitranspirantwirkstoffe, wobei die benötigte Konzentration an NPK sehr viel geringer ist als bei Verwendung von ACH.

Die pH-Werterhöhung erst vor der Applikation führt auch zur Behebung der aufgeführten Nachteile, wie Hautreizung durch zu niedrigen pH nicht stabilisierter Kieselsäuren und der in der Diskussion stehenden Toxizität von Aluminiumverbindungen.

Bevorzugt umfassen erfindungsgemäße Produkte daher neben einer NPK-haltigen Zubereitung, keine weiteren antitranspirant wirksamen Stoffe oder Zubereitungen, insbesondere keine Aluminiumsalze, insbesondere kein ACH und/oder AACH (aktiviertes Aluminiumchlorohydrat).

Wesentlicher Vorteil der erfindungsgemäßen Produkte ist darüber hinaus, dass sich gegenüber den auf Aluminiumsalzen basierenden AT-Mitteln, keinerlei Verfärbungen auf der Haut oder Kleidung zeigt. Das sogenannte Weißeln unterbleibt ebenso wie die nach mehrfachem Tragen und Waschen in direkt auf der Achselhaut aufliegenden Textilien zu beobachtenden Rückstände.

Die stabilisierten Kieselsäuren können auf einfache Weise in die zu erfindungsgemäßen Produkten geeigneten Zusammensetzungen eingearbeitet werden. Bevorzugt werden sie als NPK-Lösung den übrigen Bestandteilen der Formulierungen zugesetzt. Der Anteil der NPK-Lösung kann dabei bis zu 98% der Gesamtmenge der Formulierung ausmachen. Im einfachsten Fall werden der NPK-Suspension nur ein Verdicker und ein Parfüm zugesetzt, wobei unter Parfüm eine Abmischung umfassend ein oder mehrere olfaktorisch wahrnehmbarer Einzelsubstanzen zu verstehen ist.

Zur erfindungsgemäßen Verwendung stabilisierte Kieselsäuren lassen sich z.B. nach den folgenden Methoden im Labormaßstab herstellen:

### Methode I:

1. Herstellung einer verdünnten wässrige Na-Silikatlösung mit einem pH > 11
2. Reduktion des pH-Wertes von >11 auf <1 durch Zugabe einer entsprechenden Menge einer oder mehrerer starken Säuren innerhalb von 5-10sec.
3. ggfs Erhöhung des pH-Wertes auf einen Wert kleiner 3,5 durch Zugabe einer oder mehrerer Basen
4. Zugabe des Stabilisators

Für die in Schritt 2 zur pH-Wertreduktion eingesetzte Säure eignen sich insbesondere Mineralsäuren wie Salzsäure, Schwefelsäure oder Phosphorsäure, deren Anionen physiologisch verträglich und gut in Lösung zu halten sind. Die pH-Wertreduktion kann jedoch auch beliebige andere Säuren erfolgen, solange diese a) den pH-Wert entsprechend herabsetzen können und b) deren Salze, insbesondere Natriumsalz, physiologisch verträglich sind bzw. keine Hautreizungen verursachen. Bevorzugt wird zur pH-Wertherabsetzung Salzsäure verwendet.

Die schnelle pH-Wertreduktion ist ausschlaggebend für die erfolgreiche Bildung der NPK. Erfolgt die pH-Wertreduktion zu langsam, bilden sich sehr hochmolekulare Kieselsäuren, welche keine Antitranspirantaktivität aufweisen, bis hin zur Gelbildung.

Die Erhöhung der pH-Wertes in Schritt 3 auf einen auf einen kosmetisch akzeptablen Wert, ist optional und kann mit Natronlauge, Kalilauge oder mit schwächeren Basen erfolgen. Verwendbare Basen sind z.B.: 2-Aminobutanol, 2-(2-Aminoethoxy)ethanol, Aminoethyl Propanediol, Aminomethyl Propanediol, Aminomethyl Propanol, Aminopropanediol, Bis-Hydroxyethyl Tromethamine, Butyl Diethanolamine, Butylethanolamine, Dibutyl Ethanolamine, Diethanolamine, Diethyl Ethanolamine, Diisopropanolamine, Dimethylamino Methylpropanol, Dimethyl Isopropanolamine, Dimethyl MEA, Ethanolamine, Ethyl Ethanolamine, Isopropanolamine, Methyl Diethanolamine, Methylethanolamine, Triethanolamine, Triisopropanolamine, Tromethamine, Polyethylenimine, Tetrahydroxypropyl ethylendiamin, Ammoniak.

Die pH-Erhöhung kann ebenfalls mit Puffersystemen, welches im Sinne der Erfindung auch als Base angesehen wird, erfolgen, die entweder in wässriger Lösung oder wasserfrei zugegeben werden. Die Puffersysteme können aus den zuvor erwähnten Basen und kosmetisch akzeptablen Säuren bestehen und haben einen pH-Wert von 3 bis 11, bevorzugt 7 bis 9. Beispiele für Säuren, die zur Pufferherstellung besonders geeignet sind, sind Zitronensäure, Milchsäure, Weinsäure, Fettsäuren, Phosphorsäure, Phosphonsäuren, Polyacrylsäuren, Bernsteinsäure, Äpfelsäure, Oxalsäure, Aminosäuren.

Für die in Schritt 3 zur pH-Wertanhebung eingesetzte Base eignen sich insbesondere Alkalihydroxide, deren Kationen physiologisch verträglich und gut in Lösung zu halten sind. Insbesondere sind Natrium- und/oder Kaliumhydroxidlösung zur pH-Wertanhebung geeignet. Die pH-Wertanhebung kann vorteilhaft in Stufen erfolgen, wobei für die erste Stufe eine konzentriertere Base, z.B. 5N NaOH, eingesetzt wird und erst zur Einstellung des endgültigen pH-Wertes eine weniger konzentriertere Base, z.B. 0.5N NaOH, verwendet wird.

Vorteilhaft ist es den pH-Wert zuerst mit 5N NaOH auf pH 2 zu bringen und dann mit 0.5N NaOH weiter auf mindestens pH 3,5 zu erhöhen.

Als Stabilisatoren besonders geeignet für diese Herstellungsmethode sind Ethanol, Glycerin, 2-Propanol, PEG 8, Triethylenglycol, Urea, Oxalsäure, Hyaluronsäure, Ethylhexylglycerin, Pentaerithritol, Threitol, Erythritol, Methylphenylbutanol, Polyglyceryl 2-caprate, Decandiol

Besonders bevorzug ist eine Verwendung von Glycerin und Ethanol im Verhältnis 1:10 bis 6:10, insbesondere 5 bis 30 Gew.-% Glycerin und 30 Gew.-% EtOH bezogen auf die Gesamtmenge der in Schritt 1 hergestellten Na-Silikatlösung.

### Methode II:

1. Herstellung einer verdünnten wässrigen Na-Silikatlösung mit einem pH >11
2. Zugabe mindestens eines Stabilisators
3. Reduktion des pH-Wertes von >11 auf <1 durch Zugabe einer entsprechenden Menge einer oder mehrerer starken Säuren innerhalb von 5-10sec.
4. ggfs. Erhöhung des pH-Wertes auf Wert kleiner 3,5 durch Zugabe einer oder mehrerer Basen

Für die pH-Wertreduktion (Schritt 3) und die pH-Wertanhebung (Schritt 4) gelten dieselben Bedingungen wie zu Schritt 2 und Schritt 3 der Methode I.

Geeignete Stabilisatoren für dieses Herstellungsverfahren für stabilisierte NPK sind Sucrose, Mannose und/oder Mannit.

### Methode III:

1. Herstellung einer verdünnten Na-Silikatlösung mit einem pH >11, wobei der oder die Stabilisatoren gleichzeitig als ein Teil des Lösungsmittels darstellen
2. Reduktion des pH-Wertes von >11 auf <1 durch Zugabe einer entsprechenden Menge einer oder mehrerer starken Säuren innerhalb von 5-10sec.
3. ggfs. Erhöhung des pH-Wertes auf Wert kleiner 3,5 durch Zugabe einer oder mehrerer Basen

Für die pH-Wertreduktion (Schritt 2) und die pH-Wertanhebung (Schritt 3) gelten dieselben Bedingungen wie zu Schritt 2 und Schritt 3 der Methode I.

Geeignete Stabilisatoren für dieses Herstellungsverfahren für stabilisierte NPK ist Glycerin.

Insbesondere bei sehr hohen Glycerin-Konzentrationen von über 70%, ist die pH-Erhöhung mit 0,1 bis 0,5 M Natriumhydroxid in Glycerin vorteilhaft, da dadurch mögliche 'pH-Spitzen' verhindert werden können.

Entscheidend bei der Herstellung der NPK ist in allen drei Fällen der Schritt der exotherm verlaufenden pH-Wertreduktion. Diese pH-Wertreduktion muss sehr schnell erfolgen, so dass keine Kondensation der Monomere erfolgen kann.

Für eine erforderliche schnelle, sprunghafte und gleichmäßige Änderung des pH-Werts im Ansatzvolumen ist eine hohe Rührgeschwindigkeit mit sehr guter Durchmischung des erforderlich. Langsame oder unvollständige Durchmischung führt zur Verkürzung der Stabilität und ggfs. Minderung der AT-Leistung.

Im größeren Maßstab ist diese schnelle pH-Wertreduktion auf Grund der hohen abzuführenden Wärmemenge nicht mehr als 'Batch'-Prozess handhabbar. Großtechnisch ist die schnelle pH-Wertreduktion daher nur in einem kontinuierlichen Verfahren, bei dem der Schritt 2 (Methode I und III) bzw. Schritt 3 (Methode II) durch Zusammenführung der Reaktanden Natriumsilikatlösung und Säure in einem Durchflussreaktor erfolgt, möglich.

Die nach den Methoden I bis III erhaltenen stabilisierten NPK-Lösungen sind bei Raumtemperatur ausreichend stabil zur Verwendung in erfindungsgemäß verwendbaren Produkten. Die Stabilität nimmt mit abnehmender Temperatur zu, so dass eine Lagerung im Kühlschrank (bei 5 bis 8 Grad Celsius) vorteilhaft ist.

Entsprechend können die erfindungsgemäßen kosmetische Produkte, enthaltend in einer Kammer eine stabilisierte NPK-Zubereitung, in Form von Aerosolen, also aus Zweikammeraerosolbehältern, -quetschflaschen oder durch eine Doppelpumpenvorrichtung versprühbaren Präparaten, gestaltet sein oder in Form von mittels Roll-on-Vorrichtungen (Auftragung über Bewegungskörper, zum Beispiel Kugel oder Rolle) auftragbaren flüssigen Zusammensetzungen aus Zweikammerbehältnissen appliziert werden. Auch in Form von aus normalen Zweikammerflaschen und -behältern auftragbaren Zubeitungen.

Eine gute Methode ist auch das Verstreichen bzw. Verreiben mittels flächiger Applikatoren, die von einem Zweikammerbehältnis gespeist werden, insbesondere Applikatoren mit beflockter und/oder textiler Oberfläche, da diese eine geringe Verstopfungsneigung aufweisen.

Bevorzugte Applikationsform für die Antitranspirantien mit stabilisierter NPK sind wasserhaltige Aerosole.

Vorteilhaft, gegenüber aluminiumchlorohydrathaltigen AT-Sprays ist, das die stabilisierten NPK in der Zubereitung gelöst vorliegen und nicht vor der Verwendung des Sprays durch Schütteln resuspendiert werden müssen. Die Verstopfungswahrscheinlichkeit der Düsen wird dadurch verringert.

Weiterhin ist ein Einsatz der stabilisierten NPK in Zweikammer-Roll-ons und Zweikammerpumpsprays möglich und vorteilhaft.

Pump-Sprays bieten wie die Aerosolsprays einen berührungslosen Auftrag der AT-Zubereitung auf die Haut. Bei Pump-Sprays kann jedoch auf druckfeste Behälter verzichtet werden. Zweikammerpumpsprays lassen sich metallfrei, insbesondere aluminiumfrei gestalten.

Vorteilhaft sind zum Beispiel Behältnisse aus PE, PP oder PET, die mit einer oder zwei metallfreien Zerstäuberpumpe(n) verschlossen sind, wobei metallfrei bedeutet, dass die gepumpte Zubereitung nicht mit metallischen Bauteilen in Berührung kommt. Je nachdem ob nur eine Pumpe verwendet wird oder zwei Pumpen verwendet werden erfolgt die Zuführung über einen oder zwei Steigrohre und die Mischung der NPK-haltigen Zubereitung (Phase 1) mit der Base (Phase 2) findet vor oder nach der Pumpe/den Pumpen statt.

### Aerosolspray:

Die stabilisierten NPK (Phase 1) werden in den erfindungsgemäßen Antitranspirantformulierungen bevorzugt in einer Menge von 0,1 bis 10 Gew.-%, Gehalt an NPK bezogen auf die Gesamtmasse der Zubereitung, d.h. inklusive der ggf. vorhandenen Treibgase, eingesetzt. Insbesondere Vorteilhaft sind Konzentrationen von 0,5 bis 3 Gew.-%.

Als Wirkstofflösung wird die Summe aller Bestandteile ohne das Treibgas bezeichnet, da das Treibgas in der Regel erst bei der Abfüllung hinzukommt.

### AT-Roller:

Der Anteil an ein oder mehreren Stabilisatoren in Phase 1 kann vorteilhaft bis zu 85 Gew.-%, insbesondere bis zu 30 Gew.-%, bezogen auf die Gesamtmasse der Zubereitung, gewählt werden.

Der Anteil an SiO₂-Äquivalenten in Phase 1 wird vorteilhaft im Bereich von 0,1. Bis 6 Gew.-%, bevorzugt 0,5 bis 3 Gew.-%, bezogen auf die Gesamtmasse der Zubereitung, gewählt.

### Pumpspray:

Der Anteil an ein oder mehreren Stabilisatoren in Phase 1 kann vorteilhaft bis zu 85 Gew.-%, insbesondere bis zu 30 Gew.-%, bezogen auf die Gesamtmasse der Zubereitung, gewählt werden.

Der Anteil an NPK in Phase 1 wird vorteilhaft im Bereich von 0,1. Bis 6 Gew.-%, bevorzugt 0,5 bis 3 Gew.-%, bezogen auf die Gesamtmasse der Zubereitung, gewählt.

Im Sinne der Erfindung ist es auch, das die Zubereitungen enthaltend stabilisierten NPK (Phase 1) oder die Base (Phase 2) kosmetische Hilfsstoffe enthalten, wie sie üblicherweise in solchen Zubereitungen verwendet werden, z.B. Konservierungsmittel, Konservierungshelfer, Bakterizide, Parfüme, UV-Filter, Antioxidantien, wasserlösliche Vitamine, Mineralstoffe, suspendierte Festkörperpartikel, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Elektrolyte, organische Lösungsmittel, Alkohole, Polyole, Emulgatoren, Polymere, Schaumstabilisatoren oder Silikonderivate.

Bevorzugt werden die Zubereitungen optisch ansprechend transparent hergestellt und verwendet.

Die Phase 2 ist vorteilhaft dadurch gekennzeichnet, dass sie in Form einer wässrigen oder wässrig-alkoholischen Lösung, oder einer wasserfreien Zubereitung vorliegt.

Vorteilhaft können Phase 1 und Phase 2 auch Desodorantien zugesetzt werden.

Den üblichen kosmetischen Desodorantien liegen unterschiedliche Wirkprinzipien zugrunde. Durch die Verwendung antimikrobieller Stoffe als kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringenzien mit antimikrobiell wirksamen Stoffen in ein und derselben Zusammensetzung ist gebräuchlich.

Alle als Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexylglycerin, Phenoxyethynol, Pirocton Olamin, Koffein sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Ebenso kann ein antimikrobieller Silbercitratkomplex, wie er in der DE 202008014407 beschrieben ist, bevorzugt als desodorierender Bestandteil in Verbindung mit NPK eingesetzt werden.

Bevorzugt enthalten Phase 1 und/oder Phase 2 auch Polymere enthalten. Die Polymere stammen bevorzugt aus dem Bereich der Cellulosen und/oder der Polystyrole. Sie sind vorteilhaft hydrophob oder hydrophil modifiziert. Sie dienen der Viskositätseinstellung der Phasen und erleichtern die Pump- und Mischbarkeit, sowie das Ablauf- bzw. Verteilungsverhalten auf der Haut.

Nutzbare Polymere umfassen demnach Cellulosen, Polystyrole und/oder Alkyl-Acryl Crosspolymere und können optional den NPK-Zubereitungen zugesetzt sein.

Als übliche kosmetische Inhaltsstoffe der Phasen 1 und 2 des erfindungsgemäßen Produktes können neben Wasser, Ethanol und Isopropanol, Glycerin und Propylenglykol hautpflegende Fett- oder fettähnliche Stoffe sowie Öle, wie Ölsäuredecylester, Cetylalkohol, Cetylstearylalkohol und 2-Octyldodecanol, in den für solche Präparate üblichen Mengenverhältnissen sein, sowie schleimbildende und filmbildende Stoffe und Verdickungsmittel, z.B. Hydroxyethyl- oder Hydroxypropylcellulose, Polyacrylsäure, Polyvinylpyrrolidon und Wachse.

Aus den für kosmetische Zubereitungen bekannten Emulgatoren haben sich diese als vorteilhaft für die erfindungsgemäß verwendbaren Zubereitungen der Phase 2 herausgestellt:
Polyoxyethylen(20)-sorbitan-monolaurat, Polyoxyethylen(20)sorbitan monopalmitate, Polyoxyethylen(20)-sorbitan-monostearat , Polyoxyethylen(20)-sorbitan-monooleat, Sorbitan Trioleate, Polyglyceryl-10 Stearate, Polyglyceryl-4 Caprate, Lauryl Glucoside, Polyglyceryl-2 Dipolyhydroxylstearate, Polyglyceryl-10 Laurate, Polyglyceryl-4 Laurate, Decyl Glucoside,Propylene Glycol Isostearate, Glycol Stearate), Glyceryl Isostearate), Sorbitan Sesquioleate, Glyceryl Stearate, Lecithin, Sorbitan Oleate, Sorbitan Monostearate NF, Sorbitan Stearate, Sorbitan Isostearate, Steareth-2, Oleth-2, Glyceryl Laurate, Ceteth-2, PEG-30 Dipolyhydroxystearate, Glyceryl Stearate SE, Sorbitan Stearate (and) Sucrose Cocoate, PEG-4 Dilaurate, PEG-8 Dioleate, Sorbitan Laurate, PEG-40 Sorbitan Peroleate, Laureth-4, PEG-7 Glyceryl Cocoate, PEG-20 Almond Glycerides, PEG-25 Hydrogenated Castor Oil, Stearamide MEA, Glyceryl Stearate + PEG-100 Stearate, Polysorbate 85, PEG-7 Olivate, Cetearyl Glucoside, PEG-8 Oleate, Polyglyceryl-3 Methyglucose Distearate, PG-10 Stearate, Oleth-10, Oleth-10 / Polyoxyl 10 Oleyl Ether NF, Ceteth-10, PEG-8 Laurate, Ceteareth-12, Cocamide MEA, Polysorbate 60 NF, Polysorbate 60, PEG-40 Hydrogenated Castor Oil, Polysorbate 80, Isosteareth-20, PEG-60 Almond Glycerides, Polysorbate 80 NF, PEG-150 Laurate, PEG-20 Methyl Glucose Sesquistearate, Ceteareth-20, Oleth-20, Steareth-20, Steareth-21,Ceteth-20, Isoceteth-20, PEG-30 Glyceryl Laurate, Polysorbate 20, Polysorbate 20 NF, Laureth-23, PEG-100 Stearate, Steareth-100, PEG-80 Sorbitan Laurate.

Bevorzugt werden Glyceryl Isostearate, Glyceryl Stearate, Steareth-2, Ceteareth-20, Steareth-21, PEG-40 Hydrogenated Castor Oil, PG-10 Stearate, Isoceteth-20, Isosteareth-20 und Ceteareth-12 eingesetzt.

Als Lösungsvermittler bekannt, aber als für die erfindungsgemäß verwendbaren Zubereitungen der Phase 2 als Emulgatoren einsetzbar, sind des Weiteren bevorzugt zu wählen PEG-40 Hydrogenated Castor Oil, Polysorbate 80, Laureth-23, PEG-150 Laurate und PEG-30 Glyceryl Laurate.

Neben bzw. anstelle nichtionischer Emulgatoren sind auch kationische Emulgatoren geeignet, um stabile Formulierungen mit der erfindungsgemäßen Poylquaternium Polymeren zu erstellen. Bevorzugte geeignete kationische Emulgatoren sind zu wählen aus der Gruppe Cetrimonium Chloride, Palmitamidopropyltrimonium Chloride, Quaternium-87, Behentrimonium Chloride, Distearoylethyl Dimonium Chloride, Distearyldimonium Chloride, Stearamidopropyl Dimethylamin und/oder Behentrimonium Methosulfat.

Es ist ebenfalls vorteilhaft, den Zubereitungen der Phase 2 im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 1 bis 10 Gew.-%, bezogen auf das Gesamtgewicht der Zubereitung.

Sofern die kosmetische oder dermatologische Zubereitung der Phase 2 eine Lösung oder Emulsion oder Dispersion darstellt, können als Lösungsmittel, Konsistenzgeber und/oder hauptpflegende Wirkstoffe verwendet werden:
- Wasser oder wässrige Lösungen
- Öle, wie Triglyceride der Caprin- oder der Caprylsäure und Alkylbenzoat, vorzugsweise aber cyclische Silikonöle oder leicht flüchtige Kohlenwasserstoffe;
- -Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren; Pflanzliche Öle wie z.B. Avocadoöl, Wiesenschaumkrautöl, Olivenöl, Sonnenblumenöl, Rapsöl, Mandelöl, Nachtkerzenöl, Kokosöl. Palmöl, Leinöl, Shea Butter.
- Alkohole, Diole oder Polyole niedriger C-Zahl, sowie deren Ether, insbesondere Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.
- Hautpflegende Substanzen wie z.B. Panthenol, Allantoin, Harnstoff, Harnstoffderivate, Guanidin, Ascorbinsäure, Glycerylglucose,

Insbesondere werden Gemische der vorstehend genannten Inhaltsstoffe verwendet. Bei alkoholischen Lösungsmitteln kann Wasser ein weiterer Bestandteil sein.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Dimethylether, Distickstoffmonoxid, Kohlendioxid, Stickstoff und Druckluft sind vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bei Aerosolzubereitungen werden häufig Öle zugesetzt, die in der Wirkstofflösung mit dem Treibgas (Propan, Butan, Isobutan) mischbar sind, da ein Öl, das nicht mischbar ist, zu Niederschlägen führt, die im Glasaerosol dazu führen, dass die Wirkstoffpartikel nicht mehr aufzuschütteln sind.

Kosmetische Zubereitungen der Phase 2 können auch als Gele vorliegen, die neben einem wirksamen Gehalt am erfindungsgemäßen Wirkstoff und dafür üblicherweise verwendeten Lösungsmitteln, bevorzugt Wasser, noch organische Verdickungsmittel (Verdicker), z.B. Tamarindenmehl, Konjakmannan, Guaran, Hydroxypropylguar, Johannisbrotkernmehl, Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose, Carboxymethylcellulose oder ein Gemisch aus Polyethylenglykol und Polyethylenglykolstearat oder -distearat, enthalten. Das Verdickungsmittel ist in der Formulierung z.B. in einer Menge zwischen 0,1 und 40 Gew.-%, bevorzugt zwischen 0,5 und 25 Gew.-%, enthalten.

Ansonsten sind die üblichen Maßregeln für das Zusammenstellen von kosmetischen Formulierungen zu beachten, die dem Fachmann geläufig sind.

Es folgen vorteilhafte Ausführungsbeispiele der vorliegenden Erfindung.

Nachfolgende Beispiele erläutern die erfindungsgemäßen Zubereitungen, die zur Verminderung oder Verhinderung der Schweißbildung.

Die Zahlenangaben stellen Gewichtsanteile, bezogen auf die Gesamtmasse der Zubereitung, dar.

| | Gew.-% | Gew.-% | Gew.-% |
|---|---|---|---|
| **Phase 1 (NPK-Lösung mit pH = 1)** | **A** | **B** | **C** |
| Natriumsilikat | 11,1 | 11,12 | 11,12 |
| Wasser, entmineralisiert | 54,5 | 53,38 | 53,98 |
| Ethanol | 30 | 30 | 30 |
| HCl 37% | 4 | 4 | 4 |

| **Phase 2 (Base)** | **A** | **B** | **C** |
|---|---|---|---|
| | Gew.-% | Gew.-% | Gew.-% |
| Natriumhydroxid (NaOH) | 0,8 | | |
| Triethanolamin (TEA) | | 3,0 | |
| 2-Amino-2-methylpropanol (AMP) | | | 1,8 |
| Wasser, entmineralisiert | 20 | 20 | 20 |

Die Phase 1 und Phase 2 können in verschiedenen Verhältnissen aus dem Zweikammerpackmittel ausgetragen werden. Verhältnisse von 10:90 bis 90:10 lassen sich je nach gewähltem Packmittel durch unterschiedliche Pumpenvolumina vorbestimmen.

### Tests und Nachweise

Zum Nachweis der antitranspiranten Wirksamkeit bzw. Schweißhemmung wurde die schweißreduzierende Wirkung von 0.5M Kieselsäure gravimetrisch im Sauna-Testdesign gemessen.

### Sauna-Testdesgin:

Die axilläre Schweißmenge wird gravimetrisch ermittelt indem Baumwollpads nach einer 15min Schwitzphase in der Sauna ausgewogen werden.

Die Probanden (N=24, 12 weiblich + 12 männlich) verzichten für mind. 14 Tage vor Testbeginn auf die Verwendung von Aluminium-haltigen Produkten. Pro Achsel werden 500mg Produkt rechts/links-randomisiert und verschlüsselt aufgetragen.

6h nach Applikation der Testprodukte (10% ACH wässr. und 0.5M Kieselsäure + 30% EtOH) werden Baumwollpads in den Achseln platziert und die Schweißsekretion über einen Zeitraum von 15min in der Sauna (75°C/ 30% rel. Luftfeuchtigkeit) stimuliert.

| **Ingredient** | **"ACH"** | **"Kieselsäure"** |
|---|---|---|
| Aluminum chlorohydrate | 10% | - |
| Kieselsäure | - | 11,1% (0,5M) |
| EtOH | - | 30% |
| HCL | - | 1,7 |
| NaOH | - | 0,4 |
| H₂O | ad 100 % | ad 100 % |
| pH | 4.0 ± 0.5 | 4.0 ± 0.5 |

Die relative axilläre Schweißmenge wird auf das Schwitz-Grundniveau (Basislinie) normalisiert, das unter identischen Versuchsbedingungen vor Produktapplikation aufgenommen wurde (=100%). Die relative Schweißreduktion für die Kieselsäure beträgt im Vergleich zum unbehandelten Areal 54,1%. Die Wirksamkeit liegt damit auf dem Niveau von 10% ACH (siehe Tabelle).

| **sample** | Baselinie | nach Applikation | rel. Reduktion | Signifikanz |
|---|---|---|---|---|
| | [g] | [g] | [%] | p |
| "ACH" | 0.75 ± 0.45 | 0.35 ± 0.24 | 53,3 | <0.001 |
| "Kieselsäure" | 0.74 ± 0.48 | 0.34 ± 0.29 | 54,1 | <0.001 |

## Patentansprüche

1. Kosmetisches Produkt aufweisend ein Zweikammerbehältnis zur Applikation von kosmetischen Zubereitungen, **dadurch gekennzeichnet, dass** die erste Kammer eine Zubereitung umfassend Kieselsäuere enthält (Phase 1) und die zweite Kammer eine Zubereitung umfassend eine alkalische Verbindung (Base) enthält (Phase 2), wobei erste und zweite Kammer durch eine Barriere voneinander getrennt sind, wobei die Barriere durch Einwirkung von Kräften auf die zweite Kammer zerstört wird, wodurch der Inhalt der zweiten Kammer in die erste Kammer übertreten kann und sich Inhalt der ersten Kammer und Inhalt der zweiten Kammer vermischen und die Mischung entnommen werden kann.

2. Kosmetisches Produkt nach mindestens einem der vorhergehenden Ansprüche, **dadurch gekennzeichnet, dass** Phase 1 einen pH-Wert kleiner 2, insbesondere kleiner 1,5, aufweist.

3. Kosmetisches Produkt nach mindestens einem der vorhergehenden Ansprüche, **dadurch gekennzeichnet, dass** Phase 2 ein Parfüm enthält.

4. Kosmetisches Produkt nach einem der vorhergehenden Ansprüche, **dadurch gekennzeichnet, dass** Phase 2 einen Verdicker enthält.

5. Kosmetisches Produkt nach mindestens einem der vorhergehenden Ansprüche, **dadurch gekennzeichnet, dass** Phase 1 einen oder mehrere Stabilisator(en) gewählt aus der Gruppe Hexenol cis 3 (CAS 928-96-1), Terpineol (CAS 8000-41-7), Linalool (CAS 78-70-6), Tetrahydrolinalool (CAS 78-69-3), Triethyl Citrate (CAS 77-93-0), 2-Isobutyl-4-hydroxy-4-methyltetrahydropyran (CAS 63500-71-0), Hexyl salicylate (CAS 6259-76-3), Phenylethyl alcohol (CAS 60-12-8), 3-Methyl-5-phenyl-1-pentanol (CAS 55066-48-3), 2,6-Dimethyl-7-octen-2-ol (CAS 18479-58-8), Benzyl salicylate (CAS 118-58-1), Geraniol (CAS 106-24-1), Citronellol (CAS 106-22-9) und Ethyllinalool (CAS 10339-55-6), insbesondere aus der Gruppe Linalool (CAS 78-70-6), Benzyl salicylate (CAS 118-58-1), Geraniol (CAS 106-24-1) und Citronellol (CAS 106-22-9) enthält.

6. Kosmetisches Produkt nach mindestens einem der vorhergehenden Ansprüche, **dadurch gekennzeichnet, dass** Phase 1 einen oder mehrere Stabilisator(en) gewählt aus der Gruppe der Alkohole und Diole, insbesondere aus der Gruppe: Ethanol, 2-Propanol, PEG 8, Triethylenglycol, Methylphenylbutanol, Decandiol, Polyglyceryl-2-caprate, Oxalsäure enthält.

7. Kosmetisches Produkt nach mindestens einem der vorhergehenden Ansprüche, **dadurch gekennzeichnet, dass** Phase 1 einen oder mehrere Stabilisator(en) gewählt aus der Gruppe der Substanzen mit mindestens drei Hydroxylgruppen, insbesondere aus der Gruppe: Sucrose (Mannose, Mannit), Glycerin, Pentaerithritol, Threitol, Erythritol, Hyaluronsäure enthält.

8. Kosmetisches Produkt nach Anspruch 4, **dadurch gekennzeichnet, dass** der Verdicker gewählt wird aus der Gruppe Tamarindenmehl, Konjakmannan, Guaran, Hydroxypropylguar, Johannisbrotkernmehl, Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose, Carboxymethylcellulose oder ein Gemisch aus Polyethylenglykol und Polyethylenglykolstearat oder -distearat.

9. Kosmetisches Produkt nach mindestens einem der vorhergehenden Ansprüche, **dadurch gekennzeichnet, dass** Phase 1 und Phase 2 weniger als 0,1 Gew.-% an Aluminiumverbindungen, insbesondere weniger als 0,05 mol/l an Aluminiumionen, besonders bevorzugt frei von Aluminiumchlorohydrat ist.

10. Kosmetisches Produkt nach mindestens einem der vorhergehenden Ansprüche, **dadurch gekennzeichnet, dass** Phase 2 mindestens ein physiologisch verträgliches und/oder kosmetisches Öl und mindestens einen physiologisch verträglichen und/oder kosmetischen Emulgator enthält.

11. Verwendung eines Produktes nach mindestens einem der vorhergehenden Ansprüche, **dadurch gekennzeichnet dass** die kosmetische und/oder dermatologische Zubereitung als Aerosol oder mittels eines Bewegungskörpers topisch appliziert wird.

12. Verwendung eines Produktes nach mindestens einem der vorhergehenden Ansprüche, **dadurch gekennzeichnet dass** die kosmetische und/oder dermatologische Zubereitung mittels Verreiben bzw. Versteichen topisch appliziert wird.

## Claims

1. Cosmetic product comprising a dual chamber container for applying cosmetic preparations, **characterized in that** the first chamber contains a preparation comprising silicic acid (phase 1) and the second chamber contains a preparation comprising an alkaline compound (base) (phase 2), wherein first and second chambers are separated from one another by a barrier, wherein the barrier is destroyed by the action of forces on the second chamber, as a result of which the contents of the second chamber can pass into the first chamber and the contents of the first chamber and contents of the second chamber mix together and the mixture can be withdrawn.

2. Cosmetic product according to at least one of the preceding claims, **characterized in that** phase 1 has a pH less than 2, especially less than 1.5.

3. Cosmetic product according to at least one of the preceding claims, **characterized in that** phase 2 comprises a perfume.

4. Cosmetic product according to any of the preceding claims, **characterized in that** phase 2 comprises a thickener.

5. Cosmetic product according to at least one of the preceding claims, **characterized in that** phase 1 comprises one or more stabilizer(s) selected from the group consisting of cis-3-hexenol (CAS 928-96-1), terpineol (CAS 8000-41-7), linalool (CAS 78-70-6), tetrahydrolinalool (CAS 78-69-3), triethyl citrate (CAS 77-93-0), 2-isobutyl-4-hydroxy-4-methyltetrahydropyran (CAS 63500-71-0), hexyl salicylate (CAS 6259-76-3), phenylethyl alcohol (CAS 60-12-8), 3-methyl-5-phenyl-1-pentanol (CAS 55066-48-3), 2,6-dimethyl-7-octen-2-ol (CAS 18479-58-8), benzyl salicylate (CAS 118-58-1), geraniol (CAS 106-24-1), citronellol (CAS 106-22-9) and ethyl linalool (CAS 10339-55-6), more particularly from the group consisting of linalool (CAS 78-70-6), benzyl salicylate (CAS 118-58-1), geraniol (CAS 106-24-1) and citronellol (CAS 106-22-9).

6. Cosmetic product according to at least one of the preceding claims, **characterized in that** phase 1 comprises one or more stabilizer(s) selected from the group of alcohols and diols, more particularly from the group: ethanol, 2-propanol, PEG 8, triethylene glycol, methylphenylbutanol, decanediol, polyglyceryl-2 caprate, oxalic acid.

7. Cosmetic product according to at least one of the preceding claims, **characterized in that** phase 1 comprises one or more stabilizer(s) selected from the group of substances having at least three hydroxyl groups, more particularly from the group: sucrose (mannose, mannitol), glycerol, pentaerythritol, threitol, erythritol, hyaluronic acid.

8. Cosmetic product according to Claim 4, **characterized in that** the thickener is selected from the group consisting of tamarind flour, konjac mannan, guar gum, hydroxypropyl guar, locust bean gum flour, gum arabic, xanthan gum, sodium alginate, cellulose derivatives, preferably methylcellulose, hydroxymethylcellulose, hydroxyethylcellulose, hydroxypropylcellulose, hydroxypropylmethylcellulose, carboxymethylcellulose or a mixture of polyethylene glycol and polyethylene glycol stearate or distearate.

9. Cosmetic product according to at least one of the preceding claims, **characterized in that** phase 1 and phase 2 is less than 0.1 wt% aluminum compounds, more particularly less than 0.05 mol/l aluminum ions, particularly preferably free of aluminum chlorohydrate.

10. Cosmetic product according to at least one of the preceding claims, **characterized in that** phase 2 comprises at least one physiologically compatible and/or cosmetic oil and at least one physiologically compatible and/or cosmetic emulsifier.

11. Use of a product according to at least one of the preceding claims, **characterized in that** the cosmetic and/or dermatological preparation is applied topically as an aerosol or by means of a moving body.

12. Use of a product according to at least one of the preceding claims, **characterized in that** the cosmetic and/or dermatological preparation is applied topically by means of rubbing or spreading.

## Revendications

1. Produit cosmétique comprenant un contenant à deux chambres pour l'application de préparations cosmétiques, **caractérisé en ce que** la première chambre contient une préparation comprenant de la silice (phase 1) et la deuxième chambre contient une préparation comprenant un composé alcalin (base) (phase 2), la première et la deuxième chambre étant séparées l'une de l'autre par une barrière, la barrière étant détruite par l'action de forces sur la deuxième chambre, le contenu de la deuxième chambre pouvant ainsi passer dans la première chambre, et le contenu de la première chambre et le contenu de la deuxième chambre se mélangeant, et le mélange pouvant être soutiré.

2. Produit cosmétique selon au moins l'une quelconque des revendications précédentes, **caractérisé en ce que** la phase 1 présente un pH inférieur à 2, notamment inférieur à 1,5.

3. Produit cosmétique selon au moins l'une quelconque des revendications précédentes, **caractérisé en ce que** la phase 2 contient un parfum.

4. Produit cosmétique selon l'une quelconque des revendications précédentes, **caractérisé en ce que** la phase 2 contient un épaississant.

5. Produit cosmétique selon au moins l'une quelconque des revendications précédentes, **caractérisé en ce que** la phase 1 contient un ou plusieurs stabilisateurs choisis dans le groupe constitué par l'hexénol cis 3 (CAS 928-96-1), le terpinéol (CAS 8000-41-7), le linalool (CAS 78-70-6), le tétrahydrolinalool (CAS 78-69-3), le citrate de triéthyle (CAS 77-93-0), le 2-isobutyl-4-hydroxy-4-méthyltétrahydropyrane (CAS 63500-71-0), le salicylate d'hexyle (CAS 6259-76-3), l'alcool phényléthylique (CAS 60-12-8), le 3-méthyl-5-phényl-1-pentanol (CAS 55066-48-3), le 2,6-diméthyl-7-octén-2-ol (CAS 18479-58-8), le salicylate de benzyle (CAS 118-58-1), le géraniol (CAS 106-24-1), le citronellol (CAS 106-22-9) et l'éthyllinalool (CAS 10339-55-6), notamment dans le groupe constitué par le linalool (CAS 78-70-6), le salicylate de benzyle (CAS 118-58-1), le géraniol (CAS 106-24-1) et le citronellol (CAS 106-22-9) .

6. Produit cosmétique selon au moins l'une quelconque des revendications précédentes, **caractérisé en ce que** la phase 1 contient un ou plusieurs stabilisateurs choisis dans le groupe constitué par les alcools et les diols, notamment dans le groupe constitué par : l'éthanol, le 2-propanol, le PEG 8, le triéthylène glycol, le méthylphénylbutanol, le décanediol, le 2-caprate de polyglycéryle, l'acide oxalique.

7. Produit cosmétique selon au moins l'une quelconque des revendications précédentes, **caractérisé en ce que** la phase 1 contient un ou plusieurs stabilisateurs choisis dans le groupe constitué par les substances contenant au moins trois groupes hydroxyle, notamment dans le groupe constitué par : le sucrose (mannose, mannitol), la glycérine, le pentaérythritol, le thréitol, l'érythritol, l'acide hyaluronique.

8. Produit cosmétique selon la revendication 4, **caractérisé en ce que** l'épaississant est choisi dans le groupe constitué par la farine de tamarin, le konjac mannane, la gomme de guar, l'hydroxypropylguar, la gomme de caroube, la gomme arabique, la gomme de xanthane, l'alginate de sodium, les dérivés de cellulose, de préférence la méthylcellulose, l'hydroxyméthylcellulose, l'hydroxyéthylcellulose, l'hydroxypropylcellulose, l'hydroxypropylméthylcellulose, la carboxyméthylcellulose ou un mélange de polyéthylène glycol et de stéarate ou distéarate de polyéthylène glycol.

9. Produit cosmétique selon au moins l'une quelconque des revendications précédentes, **caractérisé en ce que** la phase 1 et la phase 2 contiennent moins de 0,1 % en poids de composés d'aluminium, notamment moins de 0,05 mol/l d'ions aluminium, de manière particulièrement préférée sont exemptes de chlorohydrate d'aluminium.

10. Produit cosmétique selon au moins l'une quelconque des revendications précédentes, **caractérisé en ce que** la phase 2 contient au moins une huile physiologiquement compatible et/ou cosmétique et au moins un émulsifiant physiologiquement compatible et/ou cosmétique.

11. Utilisation d'un produit selon au moins l'une quelconque des revendications précédentes, **caractérisée en ce que** la préparation cosmétique et/ou dermatologique est appliquée topiquement sous la forme d'un aérosol ou au moyen d'un corps mobile.

12. Utilisation d'un produit selon au moins l'une quelconque des revendications précédentes, **caractérisée en ce que** la préparation cosmétique et/ou dermatologique est appliquée topiquement par frottement ou étalement.
